# EUROPEAN PATENT APPLICATION

(11) **EP 4 431 034 A1**
(43) Date of publication of application: **18.09.2024**
(21) Application number: 23165747.9
(22) Date of filing: 30.03.2023
(51) Int. Cl.: A61B 17/50

(54) **PORTABLE FIRST-AID ANTI-ASPHYXIATION DEVICE THAT AUTOMATICALLY TAKES FOREIGN MATTER FROM RESPIRATORY AIRWAY**

(30) Priority: 13.03.2023 CN 202310245278
(71) Applicant: ChongQing Moffy Innovation Technology Co., Ltd., Chongqing, Sichuan 40114 (CN)
(72) Inventor: Lv, Liang, Chongqing, 401228 (CN); Ye, ZhengHao, Xinzhuang, 457515 (CN); Zhang, JiGuang, Xiangyang, 441002 (CN)
(74) Representative: Ipey

(57) **Abstract**

A portable first-aid anti-asphyxiation device that automatically takes foreign matter from a respiratory airway, relating to the field of medical care supplies. By pressing the trigger button, the suction force is automatically generated. The device is operated easily. The obstructions in the airway can be eliminated quickly. Since the suction force is controlled by the spring inside the device and the stops on the piston rod, the structure of the device is simple. The device generates a same force each time. The suction force may not be variable due to manual operation. Compared to the devices on the market that requires a person to operate by both hands, the device of the present disclosure is safe, can be operated easily, and can generate stable results.

## Description

### TECHNICAL FIELD

The present disclosure relates to the field of medical care supplies, and in particular to a portable first-aid anti-asphyxiation device that automatically takes foreign matter from respiratory airway.

### BACKGROUND

Data show that three children die from asphyxia every day worldwide, and an overall in-hospital mortality rate for pediatric patients who suffer asphyxia is estimated at 2.5%. Asphyxia is a leading cause of death in children, especially children under 4 years old. Asphyxia is a leading cause of brain damage in young children. Presence of a small-sized object for a few minutes may cause brain damage Surveys show that 75% of foreign matter aspiration in pediatric patients occurred in patients under 3 years old, and the majority of cases occur in the third years. Male children are more likely to aspirate foreign matter than female children. Although foreign matter aspiration is preventable, morbidity and mortality caused by foreign matter aspiration in pediatric patients remains a clinical problem.

The Heimlich manoeuvre is commonly used in emergencies. The Heimlich manoeuvre is usually performed with assistance of another person and requires the assisting person to be training specifically. Further, the age of the person to be administered by the Heimlich manoeuvre is limited. Performing the Heimlich manoeuvre improperly may result in fractures, lacerations to internal organs, and other secondary hazards. The project of the present disclosure includes three parts: a fully-automatic foreign matter device of taking foreign matter out of the throat, a first-aid emergency treatment platform, and an accident insurance claim processing center. The device is similar to the Heimlich maneuver. The Heimlich maneuver is applied by taking residual air in lungs. When the abdomen is pressed, a positively-directed air flow is generated to flush out the foreign matter that obstructed the airway, and the patient can breathe properly. For the device of the present disclosure, a mask covers the mouth and the nose of the user, and a momentary negative-directed air flow is generated in an upper airway of the patient, such that the foreign matter that obstructed the airway can be taken out. Compared to the Heimlich manoeuvre, when the device of the present disclosure is used, assistance from another person is not required. The chest or the abdomen of the patient is not pressed, and therefore, there is no risk of sternal fracture or damage to internal organs. Advantages of the device of the present disclosure includes the following. The device may be operated easily. The device can be triggered by pressing one button. Assistance from another person is not required in case of emergency. The choked patient him/herself can complete the operation. The negative pressure is constant. The negative pressure remains within a certain range constantly. The pressure may not fluctuate due to personal operations. A case that the foreign matter is unable to be taken out due to an excessively less pressure being applied and a case that an excessively large pressure is applied to cause secondary damages may be avoided.

Currently, the anti-asphyxiation devices in the art are devices that require manual operations. Although these devices can assist in clearing the foreign matter that completely blocks the upper airway in some cases, these devices have certain disadvantages. While these devices are being used, another person needs to use both hands to manually perform a suction operation on the choked person. It may be difficult for the choked person him/herself to operate the device. It may be difficult to operate the device for a choked who needs extra help, such as an infant. A suction power of the device in the art depends on a force and a speed that the user manually operates the device. Therefore, the devices in the art cannot be operating safely and conveniently, and an effect of the device is inconvenient.

The China Child Development Program (2021-2030) clearly states the need to prevent accidental choking of infants and children and requires caregivers to provide improved effective care for infants and children. The present disclosure aims to provide an advanced and easy-to-operate automatic first-aid anti-asphyxiation device to fill the domestic market.

### SUMMARY OF THE DISCLOSURE

For the shortcomings of the related art, the present disclosure provides a portable first-aid anti-asphyxiation device that automatically takes foreign matter from the respiratory airway. The device can be operated with one hand and can automatically generates a suction force by pressing a trigger button. The device can be used safely, simply and may achieve the anti-asphyxiation effect stably.

To achieve the above purpose, the present disclosure provides a portable first-aid anti-asphyxiation device that automatically takes foreign matter from the respiratory airway. The device includes a suction master member and a mask. The suction master member includes a housing, a power unit, and a piston module. The power unit drives the piston module to move inside the housing to generate a negative pressure, and the negative pressure is taken to suck out foreign matter.

In some embodiments, the housing is a tube defining a space. The tube defining the space has an inner chamber and an outer chamber. The power unit is received in the outer chamber, and the piston module is received in the inner chamber.

In some embodiments, an end of the tube is arranged with an air-intake one-way valve and two air-outlet one-way valves. The air-intake one-way valve is configured to control the inner chamber to intake air unidirectionally. The air-outlet one-way valves are configured to control the outer chamber to output air unidirectionally.

In some embodiments, the piston module includes a piston rod sealed to an inner wall of the tube. An end of the piston rod is arranged with a U-shaped seal ring, and an end of the piston rod is fixed to a press sleeve.

In some embodiments, the power unit is a spring, and the spring is disposed between the press sleeve and the tube.

In some embodiments, an outside of the suction master member is arranged with a hanging bracket.

The portable first-aid anti-asphyxiation device that automatically takes foreign matter from the respiratory airway provided in the present disclosure can achieve the following effects.

(1) The portable first-aid anti-asphyxiation device that automatically takes foreign matter from the respiratory airway is arranged with mask assemblies for various ages. A desired mask assembly may be configured for the device of the present disclosure based on demands. At the same time, the press sleeve 6 is pressed till a piston rod fixation member locks the piston rod in place, and the device can be placed at a conspicuous place in the home via the hanging bracket. In the event of a person being choked due to food or foreign matter, while a medical emergency call is being made at the first opportunity, the mask of the device of the present disclosure may be pressed against to be sealed to the skin around the nose and the mouth of the choked person. Further, a catheter inside the mask is placed against an opening of the airway in the mouth of the choked person. A button on the housing may be pressed to take out the food or the object from the airway of the choked person. In most cases, the food or the object can be successfully removed out of the throat of the patient by operating the device for once or twice. Several attempts may be made to remove the obstructing object from the throat of the patient before the medical emergency services arrive.

### BRIEF DESCRIPTION OF THE DRAWINGS

FIG. 1 is a structural schematic view of the device according to the embodiments of the present disclosure.
FIG. 2 is a cross-sectional view of a suction master member according to the embodiments of the present disclosure.
FIG. 3 is an exploded view of the device according to the embodiments of the present disclosure.

Reference numerals: 1 suction master member, 101 housing, 102 air-intake one-way valve, 103 air-outlet one-way valve, 2 mask, 3 hanging bracket, 4 piston rod, 5 U-shaped seal ring, 6 press sleeve, 7 spring, 8 mask fixing member, 9 press sleeve fixing member, 10 button, 11 button adapter, 12 button fixing member, 13 button fixing seat, 14 piston rod fixing member, 15 button spring, 16 button lock, 17 deceleration buffer, 18 buffer fixing member, 19 mask adapter, 20 mask catheter.

### DETAILED DESCRIPTION

Technical solutions in the embodiments of the present disclosure will be clearly and completely described below by referring to the accompanying drawings in the embodiments of the present disclosure. Apparently, the described embodiments are only a part of but not all of the embodiments of the present disclosure.

Examples of the embodiments are shown in the accompanying drawings. In the entire description, a same reference numeral or similar reference numerals indicate a same component or similar components or indicate components having a same function or similar functions. The embodiments described below by referring to the accompanying drawings are exemplary and are intended to be explain the present disclosure. The embodiments shall not be interpreted as limiting the present disclosure.

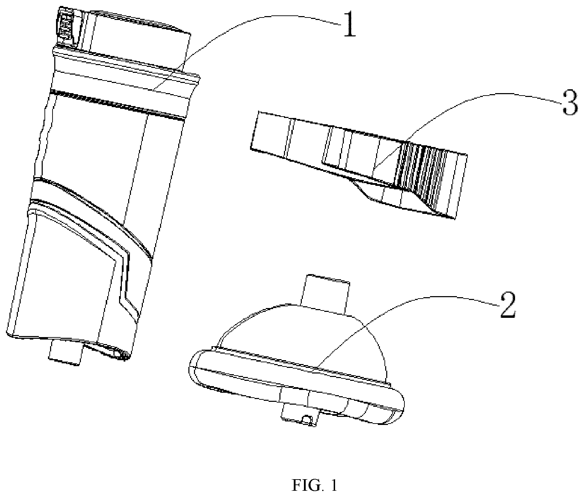

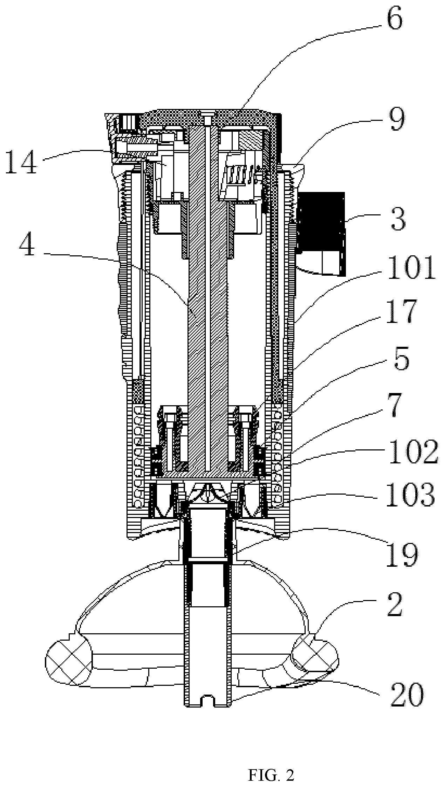

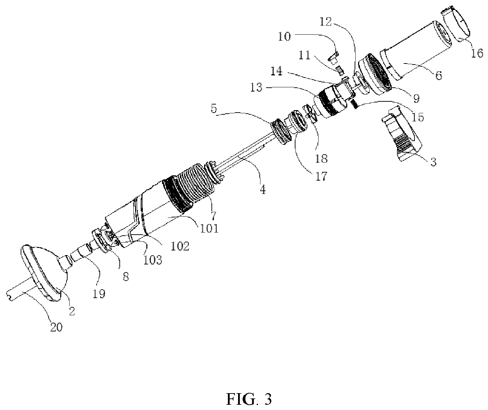

As shown in FIGs. 1 to 3, the present disclosure provides a portable first-aid anti-asphyxiation device that automatically takes foreign matter from the respiratory airway. The device includes a suction master member 1, a mask 2, and a hanging bracket 3.

The suction master member 1 includes a housing 101, a power unit, a control unit, and a piston module.

The housing 101 includes an inner chamber and an outer chamber. The outer chamber is mainly configured as the power unit and receives a spring 7 and a press sleeve 6. The inner chamber is mainly configured to allow the piston module to move. An air-intake one-way valve 102, two air-outlet one-way valves 103, and a mask fixing member 104 are fixedly arranged at a most front end of the housing 101.

The air-intake one-way valve 102 is located at a middle of the most front of the housing 101 and is above the mask 2. The air-intake one-way valve 102 is configured to prevent air from entering the mouth through the mask 2 while the piston is moving and to prevent strong air pulses from pushing food or the object further downwards. Therefore, the food or the object may not move to a deeper position of the airway of the patients.

The two air-outlet one-way valves 103 are located at two sides of the most front of the housing 101 respectively. The two air-outlet one-way valves 103 are configured to quickly evacuate air inside the inner chamber of the housing 101 when the piston rod 4 is moving towards the mask 2, allowing the inner chamber to be vacuum.

The air-intake one-way valve 102 and the air-outlet one-way valves 103 are fixed to the housing 101 by a mask fixing member 8. The mask fixing member 8 is further configured to connect a mask adapter 19.

The power unit mainly includes: a press sleeve 6, a press sleeve fixing member 9, and a spring 7.

The press sleeve 6 is configured to connect to and drive the piston rod 4 to move upwards and downwards, and at the same time, configured to compress the spring 7 to build up an elastic force. The press sleeve 6 is received in the outer chamber of the housing 101 and disposed above the spring 7.

The press sleeve fixing member 9 is configured to fixedly arrange the press sleeve 6 in the outer chamber of the housing 101.

The spring 7 is configured to serve as a power source for the piston module, driving the piston module to move to generate a negative suction force. The spring 7 is received in the outer chamber of the housing 101 and disposed below the press sleeve 6. When the piston module is located at an end of the inner chamber of the housing 101 where the mask is disposed, the spring 7 is compressed. When the piston module is located away from the end of the inner chamber of the housing 101 where the mask is disposed, the spring 7 is released, and in this case, the negative pressure is generated in the inner chamber of the housing 101.

The power unit is controlled by a control unit including: a button 10, a button adapter 11, a button fixing member 12, a button fixing seat 13, a piston rod fixing member 14, a button spring 15, and a button lock 16.

The button 10 is configured to trigger to release the piston module and is mounted on an upper outside of the housing 101.

The button adapter 11 is configured to connect the button 10 to the piston rod fixing member 14.

The button fixing member 12 is configured to fix the piston rod fixing member 14.

The button fixing seat 13 is configured to mount and fix the piston rod fixing member 14 and the button fixing member 12.

The piston rod fixing member 14 is configured to lock the piston rod 4.

The button spring 15 is configured to reset the button 10.

The button lock 16 is configured to prevent the button 10 from being mis-operated. The button 10 may be pressed only when the button lock 16 is unlocked.

The piston module includes the piston rod 4, two U-shaped seal rings 5, a deceleration buffer 17, and a buffer fixing member 18.

The piston module is configured to move reciprocally inside the housing 101. The piston module may be pushed, by pressing the press sleeve 6, to reach the end of the inner chamber of the housing 101 where the mask is disposed. At this moment, the inner chamber of the housing 101 is vacuum, the spring 7 is compressed, and the piston rod fixing member 14 locks the piston rod 4. When the button 10 is triggered, the piston rod fixing member 14 unlocks the piston rod 4, the spring 7 is released, and the piston module is instantaneously and rapidly moved away from the end of the inner chamber of the housing 101 where the mask is disposed to create the negative pressure. The suction force required to remove (sucking out) the food or the object from the airway of the choked person is rapidly generated over the airway. A plurality of stops may be arranged on the piston rod 4 to limit movement of the piston rod 4 and to control the suction force.

The piston rod 4 is configured to mount and fix the U-shaped seal rings 5, the deceleration buffer 17, and the buffer fixing member 18.

The U-shaped seal ring 5 is configured to cooperate with the piston rod 4 to create the negative pressure inside the inner chamber of the housing 101. The two U-shaped seal rings 5 are mounted facing each other, disposed at a lower end of the piston rod 4.

The deceleration buffer 17 is configured for damping and reducing noise, when the spring 7 is released due to the button 10 being triggered.

The buffer fixing member 18 is configured to fix the deceleration buffer 17.

The mask 2 includes the mask adapter 19, the mask 2, and the mask catheter 20.

The mask adapter 19 is configured to connect to the housing 101 and to fix the mask 2 and the mask catheter 10.

The mask 2 is configured to isolate inner areas of the nose and the mouth of the choked person from the outside environment, allowing the mouth of the choked person to be a sealed area. Various sized masks are available for children in various age groups.

The mask catheter 20 is configured to allow the negative pressure generated in the inner chamber of the housing 101 to be rapidly transmitted to the obstructed position of the airway. Various sized mask catheters 20 are available corresponding to the various sized masks 2.

The hanging bracket 3 is configured to fix and place the device.

When the device is being used, the press sleeve 6 presses the piston module to reach the end of the inner chamber of the housing 101 where the mask is disposed. The piston module is locked by the piston rod fixing member 14. The spring 7 in the outer chamber of the housing 101 is compressed by the press sleeve 6. The button 10 is triggered. The two air-outlet one-way valves 103 on the two sides of the front of the housing 101 are open. The air-intake one-way valve 102 in the middle of the mask 2 is closed. The air in the inner chamber of the housing 101 may be discharged from the two air-outlet one-way valves 103 on the two sides. At this moment, the inner chamber of the housing 101 is vacuumized. Since the air-intake one-way valve 102 in the middle of the mask 2 is closed, the air inside the inner chamber of the housing 101 does not enter the mouth of the user, preventing the strong air pulse from pushing the food or the object further downwards, and preventing the food or the object from reaching a deeper position of the airway of the user. When the button 10 is triggered, the button adapter 11 may push the piston rod fixing member 14 to move forward, and the spring 7 is released. At this moment, the spring 7 may spring up the press sleeve 6 instantly, and the press sleeve 6 may drive the piston module to move instantly away from the end of the inner chamber of the housing 101 where the mask is disposed. At this moment, the two air-outlet one-way valves 103 on the two sides of the front of the housing 101 are closed, and the air-intake one-way valve 102 in the middle of the mask is opened. The negative pressure is instantaneously generated inside the housing 101. The instantaneously-generated negative pressure is transmitted through the mask catheter 20 to the obstructed position of the airway of the choked user. The obstruction in the airway is fallen or removed from the airway by the suction force.

The above shows only preferred specific embodiments of the present disclosure, but the scope of the present disclosure is not limited thereto. Any equivalent substitution or modification made by any skilled person in the art based on the technical solution and the inventive concept of the present disclosure shall fall within the scope of the present disclosure.

## Claims

1. A portable first-aid anti-asphyxiation device that automatically takes foreign matter from a respiratory airway, **characterized by** comprising: a suction master member (1) and a mask (2), wherein the suction master member (1) comprises a housing, a power unit, a control unit, and a piston module, the power unit is configured to drive the piston module to move inside the housing to generate a negative pressure and take the negative pressure to suck the foreign matter out of the airway.

2. The device according to claim 1, wherein the housing is a tube (101) defining a space, the tube (101) defining the space has an inner chamber and an outer chamber, the power unit is received in the outer chamber, and the piston module is received in the inner chamber.

3. The device according to claim 2, wherein an end of the tube (101) is arranged with an air-intake one-way valve (102) and two air-outlet one-way valves (103), the air-intake one-way valve (102) is configured to control the inner chamber to intake air unidirectionally, and the two air-outlet one-way valves (103) are configured to control the outer chamber to output air unidirectionally.

4. The device according to claim 3, wherein the piston module comprises a piston rod (4) sealed to an inner wall of the tube (101), an end of the piston rod (4) is arranged with a U-shaped seal ring, and an end of the piston rod (4) is fixed to a press sleeve (6).

5. The device according to claim 4, wherein the power unit is a spring (7), and the spring (7) is disposed between the press sleeve (6) and the tube (101).

6. The device according to claim 1, wherein an outside of the suction master member (1) is arranged with a hanging bracket (3).
